Europäisches Patentamt

European Patent Office

Office européen des brevets

(19)

(11) Publication number: **0 249 405 B1**

## EUROPEAN PATENT SPECIFICATION

(12)

(45) Date of publication of patent specification: **12.08.92** (51) Int. Cl.5: **A61F 13/15**

(21) Application number: **87304980.3**

(22) Date of filing: **05.06.87**

(54) Sanitary towels.

(30) Priority: **07.06.86 GB 8613905**

(43) Date of publication of application:
**16.12.87 Bulletin 87/51**

(45) Publication of the grant of the patent:
**12.08.92 Bulletin 92/33**

(84) Designated Contracting States:
**AT BE CH DE FR GB IT LI LU NL SE**

(56) References cited:
**DE-C- 846 894**
**GB-A- 1 581 162**
**US-A- 3 805 790**
**US-A- 4 490 147**

(73) Proprietor: **SMITH & NEPHEW plc**
**2 Temple Place Victoria Embankment**
**London WC2R 3BP(GB)**

(72) Inventor: **Stringer, Karen Anne**
**21 Gwalia Grove**
**Erdington Birmingham B23 6EG(GB)**

(74) Representative: **Cole, William Gwyn, Dr.**
**Smith & Nephew p l c Corporate Patents and**
**Trade Marks Dept. Gilston Park**
**Harlow Essex CM20 2RO(GB)**

Rank Xerox (UK) Business Services

EP 0 249 405 B1

## Description

The invention relates to sanitary towels which comprise an hour-glass shaped absorbent pad, according to the preamble of claim 1.

Conventional sanitary towels comprise an absorbent pad of rectangular shape which comprises absorbent fibres such as cellulosic fibres including cotton, viscose rayon and comminuted wood pulp fibres. The rectangular shape of these towels does not conform well to the perineal area of the female and in use tend to be distorted into a bunched shape. Distortion of the towel in this manner may render the towel uncomfortable to wear and may also undesirably reduce the surface area and absorbent capacity of the towel thereby increasing the possibility of body fluid leaking to the side edges of the towel. In order to overcome some of the problems associated with conventional sanitary towels, it is known in the art, to form sanitary towels with an hour-glass shaped absorbent pad. The hour-glass shape pad provides these sanitary towels with a narrow waist region which fits between the thighs of a female and is less likely to become distorted by bunching than conventional rectangular sanitary towels. The narrow crotch region of these towels, however, has a smaller surface area for contact with the vaginal area and possible less absorbent capacity than is desirable for sanitary towels, which may lead to the leakage of body fluid to the side edges of the towel in use. US-A-3805790 discloses a sanitary napkin which comprises hour-glass shaped absorbent pad which has a thicker central portion to provide increased absorbent capacity in the crotch region of the pad. The absorbent pad disclosed in this patent, however, has a planar body facing surface which provides only a relatively small area at the waist region of the towel for positioning against the vagina. GB-A-820734 discloses a similar sanitary napkin in which the body facing surface at the waist region of the pad has a rounded surface which may assist in locating the surface adjacent to the vagina. In GB-A-1581162 there is disclosed a disposable shield for use in preventing the staining of undergarments against medications and/or daily secretions which may amount to as much as 10cc per day. Such shields comprise thin, elongate absorbent pads having a substantially planar body contacting surface. Longitudinally extending grooves may be impressed in the shield to channel fluids towards the ends.

US-A-4490147 discloses a sanitary napkin having a thickened region intermediate the ends and sides regions, the body contact surface being non-planar.

The general planar body facing surface of the hour-glass shaped sanitary napkins or towels disclosed in the above prior art patents, however, does not in use ensure contact of the surface of the narrow waist region towel against the vagina. Furthermore, there is no disclosure in these patents to a sanitary towel which has an effective means to inhibit leakage of body fluid from the narrow waist region of the pad to the side edges thereof. A sanitary towel has now been discovered which possesses these desirable properties.

Accordingly the present invention provides a sanitary towel which comprises an elongated hour-glass shaped absorbent pad having a thickened region intermediate both end regions and the sides, the body facing surface of the pad being raised with respect to the body facing surfaces of both the end regions and sides, wherein compression lines are formed longitudinally between the intermediate region and the sides and compressed areas are formed at said end regions thereby to inhibit the spread of liquid from the intermediate regions to the sides.

The shaped sanitary towel of the invention has several advantages over similar shaped towels disclosed in the prior art which comprise an absorbent pad which has a general planar body facing surface.

The raised intermediate region or portion of the absorbent pad allows the sanitary towel of the invention to make good contact with the vaginal area of a female and also increases the surface area of the towel at its narrow waist region. The raised central portion of the pad can also assist in locating the towel in its correct position. The compression lines between the central portion and the sides of the pad inhibits the leakage of liquid to the side edges of the pad.

An hour-glass shaped absorbent pad is a pad which has a shape which is symmetrical about its longitudinal central axis and which is provided with a waist so that it has a pair of opposed inwardly curved sides, a narrow centrally disposed region intermediate wider end regions.

The hour-glass shaped absorbent pad used in the invention is adapted to allow the sanitary towel of the invention in use to conform to the perineal area of a female. The pad can suitably have a length of 160 to 270mm, more suitably a length of 180 to 250mm for example a length 200 to 235mm. The front end region of the pad is preferably adapted to fit the pubic area of a female to allow the towel in use to be located in its correct position. The front end region of the pad can suitably have a width of 60 to 120mm and preferably a width of 65 to 110mm for example a width of 75 to 100mm. The narrow waist region which lies between the inwardly curved sides of the pad is adapted to fit between the thighs of a female. This narrow waist region can suitably have a width 30 to 70mm, more suitably a width of 35 to 55mm for example a width of 40 to 46mm.

The rear end region of the absorbent pad is adapted to cover the perineal of a female to the rear of the vaginal area. The rear end region of the absorbent pad can have the same width as the front end region of the absorbent pad but it is preferred that the width of the rear end region is less than that of the front end region to render the towel of the invention more comfortable when the female is seated. The rear end region of the absorbent pad can suitably have a width of 50 to 110mm and preferably have a width of 55 to 100mm, for example a width of 60 to 75mm.

The front and rear end regions of the pad can have straight ends but it is preferred that both these end regions have rounded or curved ends which preferably merge with the inwardly curved sides to form a shape which does not have pointed sides or ends.

The front and rear end regions which are wider than the narrow waist region of the pad tend to maintain the towel of the invention in a fixed position about the perineal area of a female and thus inhibit movement of the towel in use relative to the perineal area .

The raised central portion on the body facing surface of the pad extends through the narrow waist region. This raised central portion is generally elongate and adapted in size to ensure that the surface of the towel in use will make contact with the vaginal area of a female.

The central portion provides the thickest portion of the pad. Such a central portion can suitably have a thickness of 10 to 40mm and preferably a thickness of 20 to 35mm.

This portion can have a planar surface but preferably has a rounded or convex surface in the width direction of the pad and a flat or shallow convex curved surface in the length direction of the pad to provide the central portion of the towel with a surface which can lie comfortably against the vaginal area of a female.

The central portion can be in a position on the absorbent pad in which its transverse central axis coincides with that of the waist region of the pad but it is preferred that this central portion is located closer to one end region than the other to provide that region of the pad with more absorbent capacity. In a sanitary towel for normal use the central portion can favourably be located closer to the rear end region of the pad. However, in a sanitary towel for night use the central portion can advantageously be located towards front end region of the pad.

The absorbent pad used in the invention preferably tapers in thickness from the thicker raised central portion to the thinner end regions to provide the pad with a relatively smooth profile in longitudinal direction. The edges of the pad at the back end regions can be relatively thick for example a half of the thicknes of the central portion but it is preferred that the edges of the pad at both end regions are relative thin for example 1 to 2mm thick so that the tapered end regions of the towel are less conspicious when worn.

The absorbent used in the invention will normally comprise an absorbent fibrous material.

The absorbent pad has a compression lines formed intermediate of the sides and the central portion of the pad to inhibit the spread of liquid to the side edges of the pad. Compression lines provides the absorbent pad with linear areas of increased fibre density which absorb and wick aqueous liquids such as body fluids more rapidly than an area of less fibre density. Aqueous liquids such as body fluids contacting the compression lines on both sides of the raised elongate portion of the pad will tend to be absorbed rapidly or wicked along the compression line before being absorbed into the absorbent pad thereby inhibiting the spread of the liquid to the side edges of the pad and therefore also any leakage of liquid from the pad resulting therefrom.

The compression line at both sides of the central portion can advantageously extend into one or preferably both end regions of the pad to distribute liquid to these regions. It is preferred, however, that the compression lines do not extend to the end or ends of the pad where they may cause leakage of liquid from these ends. Each compression line at both sides of the central portion can advantageously be connected to a compressed area at one or preferably at both end regions of the pad to aid absorption of liquid at these end regions. An end region or both end regions can have more than one compressed area for example two compressed areas each of which is connected to a separate compression line so that each end of each compression line is connected to a separate compression area.

The compressed area at the end region of the pad can advantageously be a compression line arranged to define endless path such as a closed shape for example a circle or ellipse which confines the spread of liquid to the area enclosed by the compression line. The compression line of closed shape can advantageously extend substantially across and end region of the pad to provide an area which inhibits the spread of liquid to the edge of the end region.

In another aspect the sanitary towel of the invention has at least one compression line formed in the body facing surface which extends from the central portion to at least one end region of the pad to spread liquid from said central portion to said end region of the pad.

The compression line or lines preferably extend from the central portion to an end region at both ends of the pad. Such compression lines can be connected to a closed shape compressed area as hereinbefore described.

The compression line or lines of the absorbent pad used in the invention will normally be a linear depressed area of the pad such as a channel with for example a U or V shape. A compression line in the form of a channel on both sides of the central portion of the pad can be on either surface of the pad but is preferably that the channel is on the body facing surface of the pad. The channel shape of a compression line on the body facing surface of the pad can provide a further barrier to liquid spreading from the central portion to the side edges of the pad. A compression line can also be in the form of two channels in line with each other on opposed surfaces of the pad. Compression lines in the form of a channel extending from the central portion or at the end regions of the pad, however, will normally be on the body facing surface of the pad.

The compression line in the form of a channel on a surface of the absorbent pad can suitably have a depth of 5 to 20mm.

The absorbent pad used in the invention will normally have a liquid pervious cover layer on its body facing side and a liquid impermeable barrier layer on its side opposite the body facing side. The cover and barrier layers preferably extend beyond the edge absorbent pad to form the peripheral edge of the sanitary towel of the invention. The inner surfaces of the cover and barrier layers can advantageously be joined at the peripheral edge of the towel for example by adhesive or heat sealing to provide a barrier to liquid wicking along the cover layer to the edge of the towel.

The cover and barrier layers used in the invention can be any of the materials normally used for this purpose in conventional sanitary towels. Preferred materials for the cover layers used in the invention include non-woven fabrics and plastics nets. Preferred materials for the barrier layers used in the invention include plastics films such as low density polyethylene film.

The absorbent pad used in the invention can comprise any of the absorbent fibrous materials normally used in the absorbent pads of conventional sanitary towels including cellulosic fibres such as comminuted wood pulp fibres, cotton fibres, viscose rayon fibres and highly absorbent polymers known as super-absorbent polymers. Such highly absorbent polymers can be in particulate form such as powder or fibre or in the form of a layer for example an impregnated or coated layer. Highly absorbent polymer in particulate or layer form can conveniently be provided within or at the bottom surface of the absorbent pad.

The sanitary towel of the invention can have a fleece layer for example rayon fleece layer between the cover layer and the body facing surface of the pad to provide a soft surface for the towel.

In preferred embodiments of the invention the barrier layer is bonded to the surface of the absorbent pad opposite the body facing surface of the pad for example by adhesive in the form of one or more spaced lines of extending in the length direction of the towel to stabilise the sanitary towel structure.

The outer surface of the barrier layer of the towel can have an adhesive layer for example in the form of a strip or pair of parallel strips of pressure sensitive adhesive which extend in the length direction of the towel and are covered with a release protector to enable the sanitary towel of the invention to be attached to a supporting garmet.

The absorbent pad used in the invention can be formed by forming an hour-glass shaped pad from a preformed strip of fibrous material for example by stamping method and then forming the raised elongate central portion by moulding the pad under pressure using a suitable pressure moulding apparatus. In a alternative method the shaped pad can be formed by depositing for example by air laying or vacuum forming the fibrous material into a mould of suitable shape and if necessary consolidating the material under pressure.

The compression lines and the compressed areas in the shaped pad can be then formed by a conventional embossing or stamping method using a pressure device such as a roller, plate or die having projections of the appropriate pattern, shape and size. The compression lines at both sides of the central elongate portion can advantageously be formed during or after moulding the pad shape by a vacuum forming process.

The shaped absorbent pad can then be formed into a sanitary towel of the invention using a conventional method.

The invention will now be illustrated by reference to the following drawings in which:

Fig 1     is plan view of a sanitary towel of the invention with its body facing upwards.

Fig 2     is cross-section of the sanitary towel of Fig 1 along the line A - A.

Fig 3     is cross-section of the sanitary towel Fig 1 along the liens B - B.

Fig 4     is a plan view of another sanitary towel of the invention.

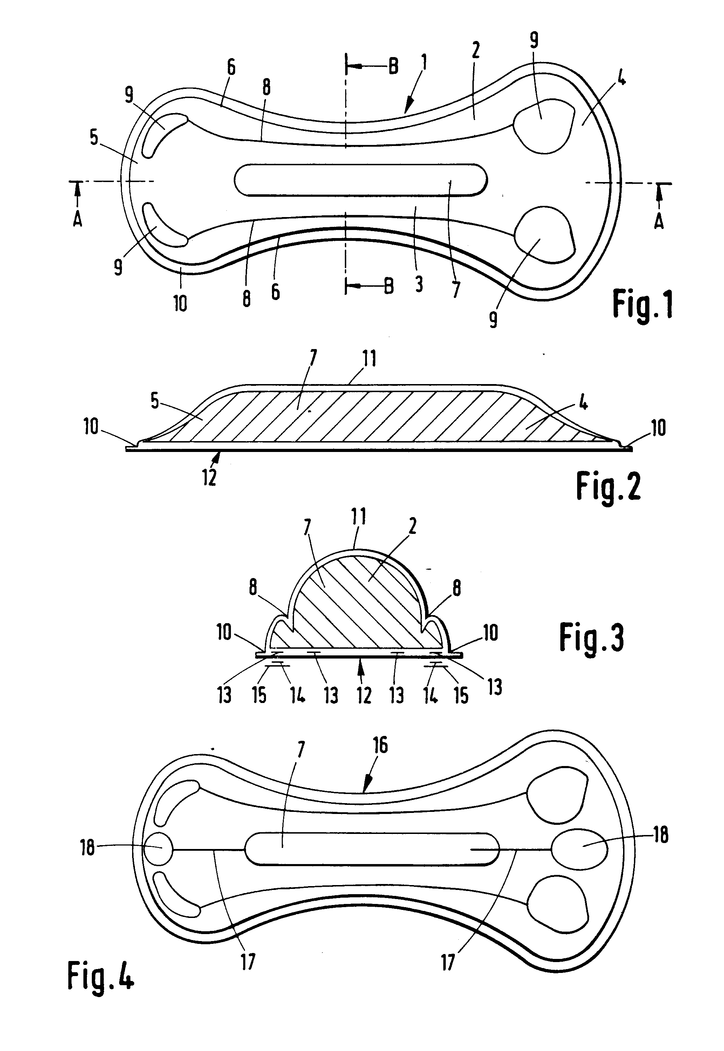

Fig 1 shows a sanitary towel (1) of the invention which comprises an elongate hour-glass shaped absorbent pad (2) which has a narrow waist region (3) a front and region (4), a rear end region (5) and inwardly curved sides (6). The absorbent pad (2) has on its body facing surface a raised elongate central portion (7) and a compression line (8) on both sides of the central portion (7). The compression lines (8) extend to the front end and rear end regions (4,5) of the pad (2) where they are connected to compressed areas (9) in the form of compression lines of closed approximately circular shape. The front end region (4) of the absorbent pad (2) in Fig 1 is wider than the rear end region (5) and the raised elongate central portion (7) is shown slightly displaced towards the rear end region (5) of the pad. The sanitary towel (1) shown in Fig 1 has a projecting peripheral edge (10).

Fig. 2 shows longitudinal cross-section of the sanitary towel shown in Fig 1 along the lines A - A. The absorbent pad (2) shown in Fig 2 has a thick raised elongate central portion (7) of substantially flat profile in the longitudinal direction and end regions (4,5) which taper in thickness from the central portion(7) to the ends of the pad (2). The absorbent pad (2) has a liquid pervious cover layer (11) on its body facing surface and liquid impermeable barrier layer (12) on its opposite surface which are joined at the peripheral edge (10) of the towel.

Fig 3 shows a transverse cross-section of the sanitary towel shown in Fig 1 along the line B-B. The absorbent pad shown in Fig 3 has compression lines (8) in the form of channels in its body facing surface and is attached on its outer surface to the barrier layer (12) by four strips of adhesive (13). Sanitary towel (1) shown in Fig has a pair of adhesive strips (14) which are covered by protector strips (15) on the outer surface to the barrier layer to adhere the towel to a supporting garment surface.

Fig 4 shows a sanitary towel (16) similar to that showing Fig 1 to 3 which has in addition compression lines (17) which extend from the central portion (7) to compressed areas (18) in the end regions of the pad (2).

The raised elongate central position (7) on the body facing surface of the absorbent pad (2) provides the sanitary towel of the invention with a surface which will make contact with the vaginal area of a female when the towel is worn.

The surface profile of the central portion (7) provides a greater surface area than the planar surface used in prior art sanitary towels and also assists in positioning the towel correctly over the perineal area of a female

Compression lines (8) in the form of channels in hibit in use the spread of liquid from the central portion (7) to the side edges of the pad (2) thereby reducing the possibility of leakage of fluid from these side edges which may stain a supporting garmet.

## Claims

1. A sanitary towel (1) comprising an elongate hour-glass shaped absorbent pad (2) having a thickened region (7) intermediate both the end regions (4, 5) and the sides, the body facing surface of the pad being raised with respect to the body facing surfaces of both the end regions and sides, characterized in that compression lines (8) are formed longitudinally between the intermediate region and sides and compressed areas are formed at said end regions thereby to inhibit spread of liquid from the intermediate region to the sides.

2. A towel is claimed in claim 1 wherein the compression lines extend to at least one end region.

3. A towel is claimed in claim 2 wherein each compression line extends to one of the compressed areas (9).

4. A towel as claimed in claim 3 wherein each end of each compression line is connected to a separate compressed area.

5. A towel is claimed in any one of the preceding claims wherein at least one compression line is formed centrally in the body facing surface extending from the intermediate region to at least one end region.

6. A towel as claimed in claim 5 wherein the compression line extends to one of the compressed areas.

7. A towel as claimed in any one of claims 3 to 6 wherein the compressed area is a compressed line arranged to define an endless path.

8. A towel as claimed in any one of the preceding claims wherein the thickened, raised intermediate region is located closer to one end than to the other.

9. A towel as claimed in any one of the preceding claims wherein the thickness of the pad decreases gradually from the intermediate region towards each end region.

10. A towel as claimed in any one of the preceding claims which comprises a liquid pervious cover layer (11) on its body facing side and a liquid impermeable layer (12) on its opposite side wherein the layers extend beyond the absorbent pad and are joined together to form the peripheral edge (10) of the towel.

## Revendications

1. Serviette hygiénique (1) comprenant un tampon absorbant (2) de forme allongée avec des extrémités arrondies et une zone centrale étrécie, ayant une région épaissie (7) intermédiaire entre les deux régions terminales (4, 5) et les côtés, la surface faisant face au corps du tampon étant surélevée par rapport aux surfaces faisant face au corps des deux régions terminales et des côtés, caractérisée en ce que des lignes de compression (8) sont formées longitudinalement entre la région intermédiaire et les côtés et que des zones comprimées sont formées dans ces régions terminales de façon à empêcher un étalement de liquide depuis la région intermédiaire vers les côtés.

2. Serviette suivant la revendication 1, dans laquelle les lignes de compression se prolongent jusqu'à une région terminale au moins.

3. Serviette suivant la revendication 2, dans laquelle chaque ligne de compression se prolonge jusqu'à l'une des zones comprimées (9).

4. Serviette suivant la revendication 3, dans laquelle chaque extrémité de chaque ligne de compression est raccordée à une zone comprimée distincte.

5. Serviette suivant l'une quelconque des revendications précédentes, dans laquelle une ligne de compression au moins est formée au centre dans la surface faisant face au corps et se prolonge depuis la région intermédiaire jusqu'à une région terminale au moins.

6. Serviette suivant la revendication 5, dans laquelle la ligne de compression se prolonge jusqu'à l'une des zones comprimées.

7. Serviette suivant l'une quelconque des revendications 3 à 6, dans laquelle la zone comprimée est une ligne comprimée disposée de façon à définir un chemin sans fin.

8. Serviette suivant l'une quelconque des revendications précédentes, dans laquelle la région intermédiaire, surélevée. épaissie, est située plus près d'une extrémité que de l'autre.

9. Serviette suivant l'une quelconque des revendications précédentes, dans laquelle l'épaisseur du tampon diminue graduellement depuis la région intermédiaire vers chaque région terminale.

10. Serviette suivant l'une quelconque des revendications précédentes, qui comprend une couche (11) de couverture perméable aux liquides sur son côté faisant face au corps et une couche (12) imperméable aux liquides sur son côté opposé dans laquelle les couches se prolongent au-delà du tampon absorbant et sont réunies pour former le bord périphérique (10) de la serviette.

## Patentansprüche

1. Eine Monatsbinde (1) mit einem langgestreckten absorbierenden Kissen (2) in Form einer Eieruhr, das einen verdickten Bereich (7) zwischen beiden Endbereichen (4, 5) und den Seiten aufweist, wobei die dem Körper zugewandte Oberfläche des Kissens in besug auf die dem Körper zugewandten Oberflächen sowohl der Endbereiche als auch der Seiten erhöht ist, dadurch gekennzeichnet, daß Kompressionslinien (8) in Längerichtung zwischen dem Zwischenbereich und den Seiten und zusammengepreßte Bereiche an den besagten Endbereichen gebildet sind, um das Ausbreiten von Flüssigkeit vom Zwischenbereich zu den Seiten zu verhindern.

2. Binde nach Anspruch 1, bei welcher sich die Kompressionslinien bis zu wenigstens einem Endbereich erstrecken.

3. Binde nach Anspruch 2, bei welcher jede Kompressionslinie sich bis zu einem der zusammengepreßten Bereiche (9) erstreckt.

4. Binde nach Anspruch 3, bei welcher jedes Ende jeder Kompressionslinie mit einem getrennten zusammengepreßten Bereich verbunden ist.

5. Binde nach einem der vorhergehenden Ansprüche, bei welcher wenigstens eine Kompressionslinie mittig in der dem Körper zugewandten Oberfläche gebildet ist, die sich vom Zwischenbereich bis zu wenigstens einem Endbereich erstreckt.

6. Binde nach Anspruch 5, bei welcher sich die Kompressionslinie bis zu einem der zusammengepreßten Bereiche erstreckt.

7. Binde nach einem der Ansprüche 3 - 6, bei welcher der zusammengepreßte Bereich eine zusammengepreßte Linie ist, die so angeordnet ist, daß sie eine endlose Bahn darstellt.

8. Binde nach einem der vorhergehenden Ansprüche, bei welcher der verdickte erhöhte Zwischenbereich näher an dem einen Ende liegt als an dem anderen.

9. Binde nach einem der vorhergehenden Ansprüche, bei welcher die Dicke des Kissens vom Zwischenbereich zu jedem Endbereich hin allmählich abnimmt.

10. Binde nach einem der vorhergehenden Ansprüche, welche eine flüssigkeitsdurchlässige Abdeckschicht (11) auf ihrer dem Körper zugewandten Seite und eine flüssigkeitsundurchlässige Schicht (12) auf ihrer entgegengesetzten Seite aufweist, wobei die Schichten sich über das absorbierende Kissen hinaus erstrecken und verbunden sind, um den Umfangsand (10) der Binde zu bilden.

Fig.1

Fig.2

Fig.3

Fig.4